# EUROPEAN PATENT APPLICATION

(11) **EP 4 480 487 A1**
(43) Date of publication of application: **25.12.2024**
(21) Application number: 22927488.1
(22) Date of filing: 26.12.2022
(51) Int. Cl.: A61K 31/717, A61K 31/718, A61K 38/48, A61P 7/04, A61P 17/02, A61L 24/04, A61L 24/00, A61L 24/02, C08L 1/08, C08L 3/04

(54) **PHARMACEUTICAL COMPOSITIONS FOR HEMOSTASIS AND WOUND HEALING IN GASTROINTESTINAL TRACT**

(30) Priority: 15.02.2022 KR 20220019745; 22.03.2022 KR 20220035054
(71) Applicant: CGBIO Co.,Ltd., Seoul 04349 (KR)
(72) Inventor: YOON, Hong Sun, Yongin-si Gyeonggi-do 17117 (KR); KWON, Kyeong Nan, Uiwang-si Gyeonggi-do 16080 (KR)
(74) Representative: Viering, Jentschura & Partner mbB Patent- und Rechtsanwälte
(86) International application number: PCT/KR2022/021261
(87) International publication number: WO 2023/158092

(57) **Abstract**

Provided are a pharmaceutical composition for hemostasis in the gastrointestinal tract and a pharmaceutical composition for wound healing in the gastrointestinal tract. The pharmaceutical compositions, according to one embodiment, may comprise: a first component comprising a water-soluble copolymer attachable to gastrointestinal mucosal tissue; a second component comprising a moisture-absorbing material; and thrombin. The pharmaceutical composition for hemostasis in the gastrointestinal tract and the pharmaceutical composition for wound healing in the gastrointestinal tract, according to one embodiment of the present invention, have the first component and the second component exhibiting a synergistic effect with thrombin, and thus have more excellent hemostasis and wound healing effects than existing commercially available hemostatic agents, have more excellent hemostasis and wound healing effects even than when thrombin is mixed into other existing commercially available hemostatic agents, and have more excellent hemostasis and wound healing effects even than when thrombin is used alone.

## Description

### [Technical Field]

The present disclosure relates to a pharmaceutical composition for hemostasis and wound healing in the gastrointestinal tract, and to a pharmaceutical composition for hemostasis and wound healing in bleeding gastrointestinal ulcers that is effectively administered into the gastrointestinal tract, and a pharmaceutical preparation containing the same. Meanwhile, this patent application claims priority with respect to Korean Patent Application No. 10-2022-0019745 submitted to the Korean Intellectual Property Office on February 15, 2022, and Korean Patent Application No. 10-2022-0035054 submitted to the Korean Intellectual Property Office on March 22, 2022, and the disclosures of the patent applications are incorporated into this specification by reference.

### [Background Art]

Gastrointestinal bleeding is a disease commonly encountered in clinical practice, and approximately 50% of gastric ulcer patients have been diagnosed with bleeding gastric ulcers. 80% or more of gastrointestinal bleeding occurs in the upper gastrointestinal tract, and upper gastrointestinal bleeding is a disease in which bleeding occurs from lesions in the esophagus, stomach, and duodenum, causing vomiting of blood or bloody stool. For upper gastrointestinal bleeding, the bleeding lesion can be identified in 90% or more of cases through endoscopy. Recently, a polypectomy of the stomach or colon, mucosal resection for the treatment of early stomach or colon cancer, and therapeutic endoscopy procedures have variously been performed, and there are cases where bleeding occurs during or after these procedures, requiring emergency surgery or even leading to death.

Hemostasis using an endoscope has recently been used as a treatment method for gastrointestinal bleeding. As such endoscopic hemostasis methods, methods such as local injection of hypertonic saline, epinephrine or alcohol directly into the ulcer, methods using coagulation therapy using electric heat, argon or laser, and hemostasis using clips have been used. However, the treatment method of the conventional technology described above is a method of reducing bleeding by injecting a liquid medicine around the blood vessel where the ulcer is exposed to compress the blood vessel or ligating the blood vessel itself, and ulcers at the wound site are left alone in a state where the mucous membrane has fallen off. Therefore, cases in which hemostasis is not achieved despite endoscopic hemostasis procedures frequently occur, and studies have reported that only about 70 to 80% of hemostasis is achieved when conventional endoscopic hemostasis methods are used. In addition, even after hemostasis, rebleeding occurs 3 to 4 days later in 20 to 25% of cases.

Rebleeding is bleeding occurring in the blood vessel before tissue regeneration at the ulcer site occurs to treat the ulcer, and it has a problem that cannot be solved with conventional endoscopic treatment methods of performing hemostasis while maintaining the state that the mucous membrane of the ulcer is removed and exposed. In other words, existing endoscopic treatment methods have the disadvantage that slow rate of treating the wound area and the possibility of rebleeding coexist. In order to improve such a problem, an in-body hemostatic agent that induces hemostasis and minimizes the possibility of rebleeding by dispersing and coating a coating agent in the form of a polymer solution on the ulcer area in the body through an endoscope, and a method of applying the same have been disclosed in Korean Patent Publication No. 10-2006-0040329.

Meanwhile, in order to speed up the treatment speed of the wound area and minimize the possibility of rebleeding, a multifunctional preparation is required that not only can prevent bleeding (i.e. hemostasis), but also effectively protect the wound area and furthermore prevent adhesion. That is, there is a need for a preparation that can be administered (injected) into the wound area of the gastrointestinal tract through an endoscope catheter and play the role of protecting the wound, preventing bleeding, and/or preventing adhesion. For topical application within the gastrointestinal tract, it must have appropriate viscosity conditions and appropriate mucosal adhesion, and form a physical barrier to the target area to provide physical bleeding prevention.

To prevent adhesion at the procedure area, preparations mainly in the form of gel or sol-gel have been developed in the past. Low-viscosity gel-type preparations have a lot of loss when applied to the upper gastrointestinal tract, which limits the formation of a physical barrier, while high-viscosity gel-type preparations have excellent mucosal adhesion, but require a high-pressure chemical liquid ejection device, which also has a demerit of entailing considerable loss. The sol-gel type preparation is a preparation that gels at a certain temperature (body temperature) in a sol state before injection into the body, and since the sol-gel transition section is small, there is a problem of gelation within the catheter before application into the body, and there is also a considerable amount of loss involved.

To solve such problems, various conventional hemostatic agents and wound healing agents have been developed, but in clinical sites, there is still a need for the development of pharmaceutical compositions and pharmaceutical preparations capable of more rapid hemostatic action and prevention of rebleeding.

### [Disclosure]

### [Technical Problem]

The present disclosure is intended to provide a pharmaceutical preparation for hemostasis and wound healing in bleeding gastrointestinal ulcers that is effectively administered into the gastrointestinal tract, and provide a pharmaceutical composition that can reduce the hemostatic effect and wound healing time and significantly improve its effect compared to conventional commercially available wound healing agents such as hemostatic agents and wound dressings.

Further, a pharmaceutical preparation prepared from the composition according to one embodiment of the present disclosure is allowed to be formulated in a powder form, thereby intending to provide a pharmaceutical preparation which makes it easy to pass through a catheter and be applied by having appropriate fluidity and can minimize the loss of the pharmaceutical preparation by forming a barrier through instantaneous gelation at the application site.

### [Technical Solution]

To solve the problems as described above, the present disclosure provides a pharmaceutical composition for hemostasis and a pharmaceutical composition for wound healing in the gastrointestinal tract. In one embodiment, the pharmaceutical composition may include a first component composed of a water-soluble copolymer that is attached to gastrointestinal mucosal tissue, a second component composed of a water-absorbing material, and thrombin.

### [Advantageous Effects]

The pharmaceutical preparation prepared from the composition according to one embodiment of the present disclosure can reduce the hemostatic effect and wound healing time and significantly improve its effect compared to conventional commercially available wound healing agents such as hemostatic agents and wound dressings.

In addition, the pharmaceutical preparation prepared from the composition according to one embodiment of the present disclosure can achieve a remarkable synergistic effect in hemostatic effect and wound healing effect compared to the case of using each of the first component and the second component and the case of using only thrombin by applying a pharmaceutical composition in which the first component, the second component, and thrombin are mixed.

In addition, the pharmaceutical preparation prepared from the composition according to one embodiment of the present disclosure is allowed to be formulated in a powder form, thereby making it easy to pass through a catheter and be applied by having appropriate fluidity and enabling the loss of the pharmaceutical preparation to be minimized by forming a barrier through instantaneous gelation at the application site.

### [Description of Drawings]

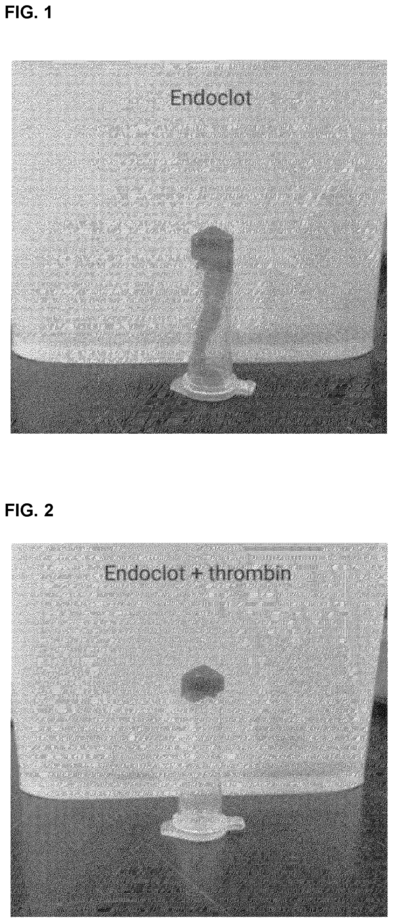

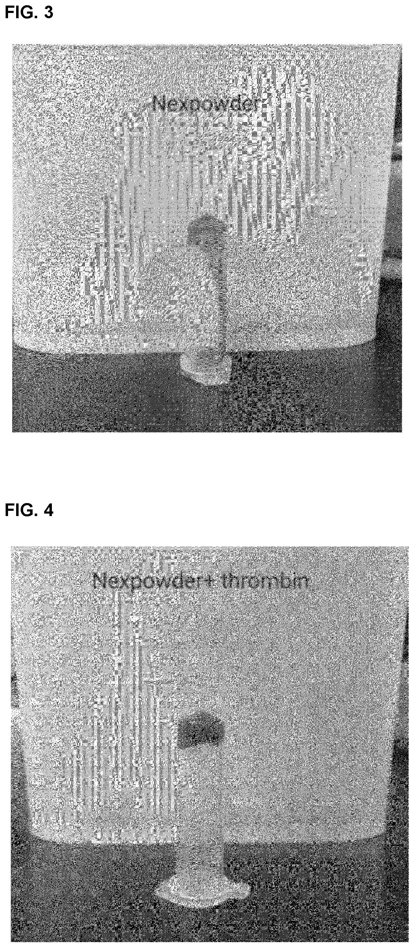

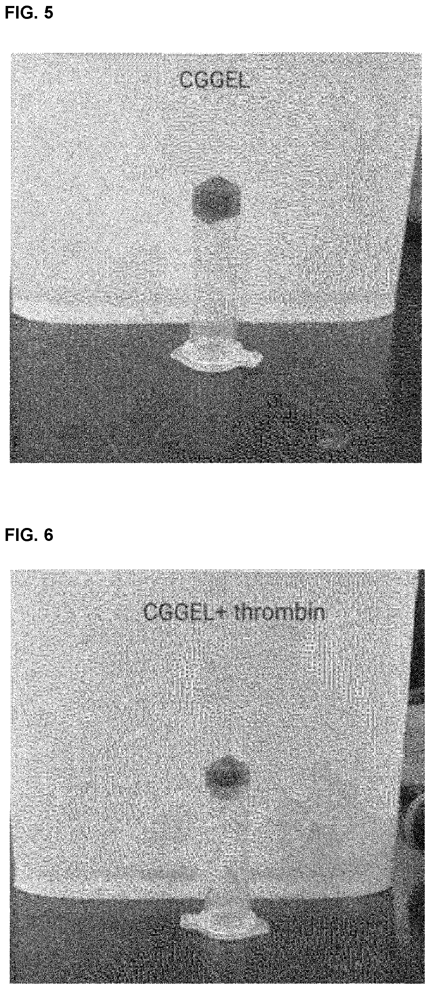

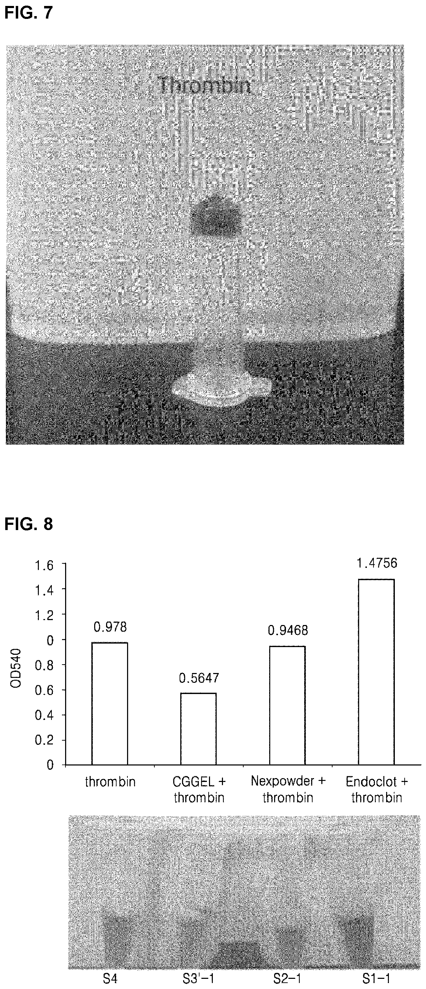

FIGS. 1 to 7 show results of blood clotting time measurement experiments performed according to Example 1-1 of the present disclosure, where FIG. 1 shows a result of the experiment performed on the S1 sample, FIG. 2 shows a result of the experiment performed on the S1-1 sample, FIG. 3 shows a result of the experiment performed on the S2 sample, FIG. 4 shows a result of the experiment performed on the S2-1 sample, FIG. 5 shows a result of the experiment performed on the S3 sample, FIG. 6 shows a result of the experiment performed on the S3'-1 sample, and FIG. 7 shows a result of the experiment performed on the S4 sample.

FIG. 8 shows results of measuring the degree of red blood cell hemolysis performed according to Experimental Example 1-2-1 of the present disclosure.

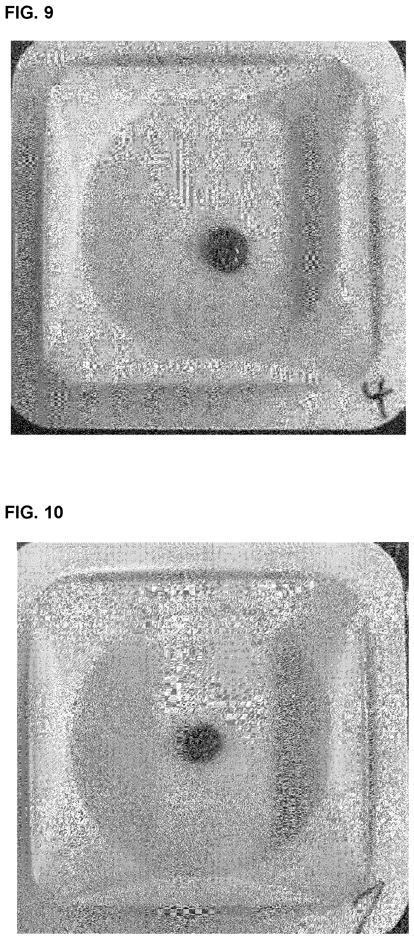

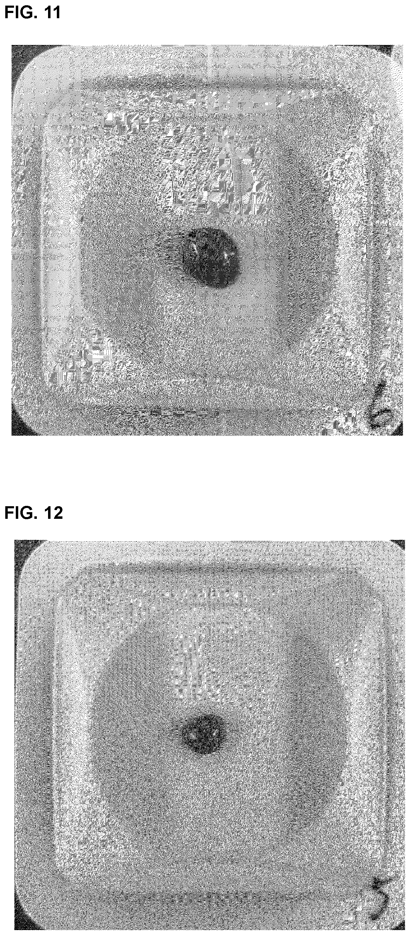

FIGS. 9 to 12 show results of blood clot weight measurement performed according to Experimental Example 1-2-2 of the present disclosure, where FIG. 9 shows a result of the experiment performed on the sample S 1-1, FIG. 10 shows a result of the experiment performed on the sample S2-1, FIG. 11 shows a result of the experiment performed on the sample S3'-1, and FIG. 12 shows a result of the experiment performed on the sample S4.

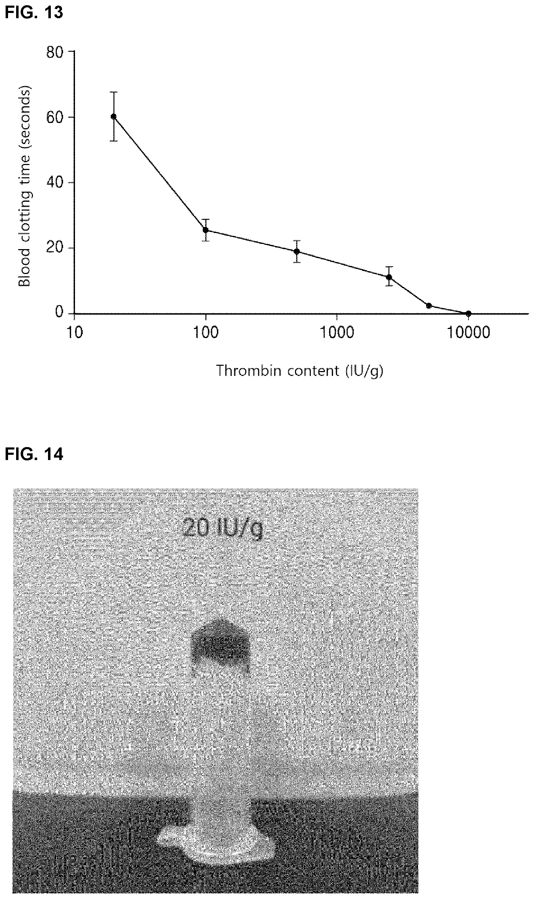

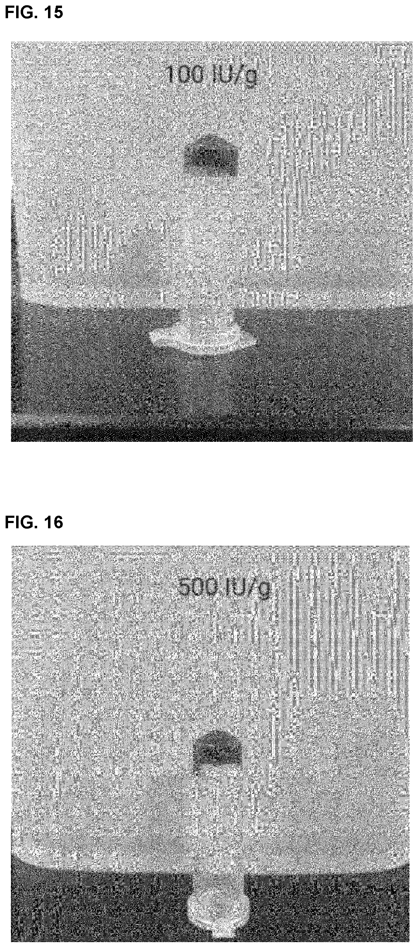

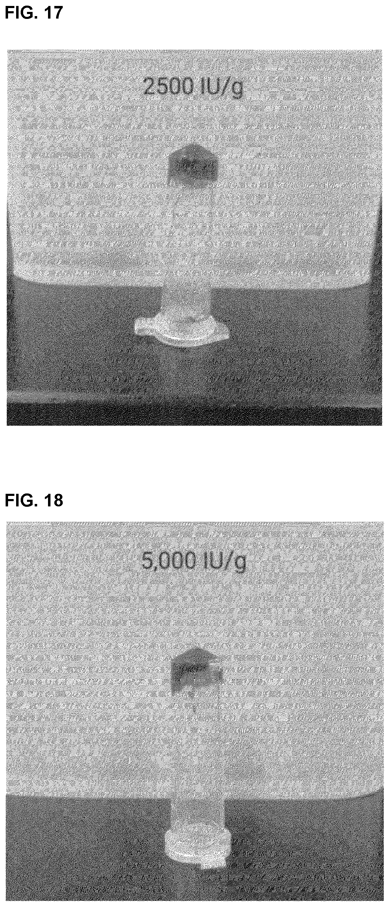

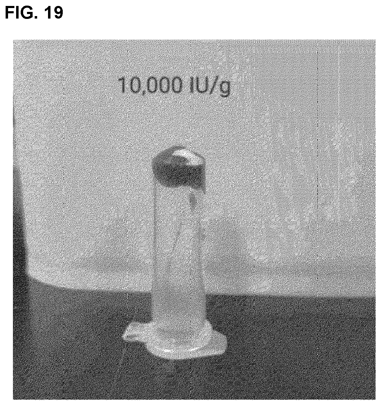

FIGS. 13 to 19 show results of blood clotting time measurement experiments performed according to Experimental Example 1-3 of the present disclosure, where FIG. 14 shows a result of the experiment performed on the S5 sample, FIG. 15 shows a result of the experiment performed on the S6 sample, FIG. 16 shows a result of the experiment performed on the S7 sample, FIG. 17 shows a result of the experiment performed on is the S8 sample, FIG. 18 shows a result of the experiment performed on the S9 sample, and FIG. 19 shows a result of the experiment performed on the S10 sample.

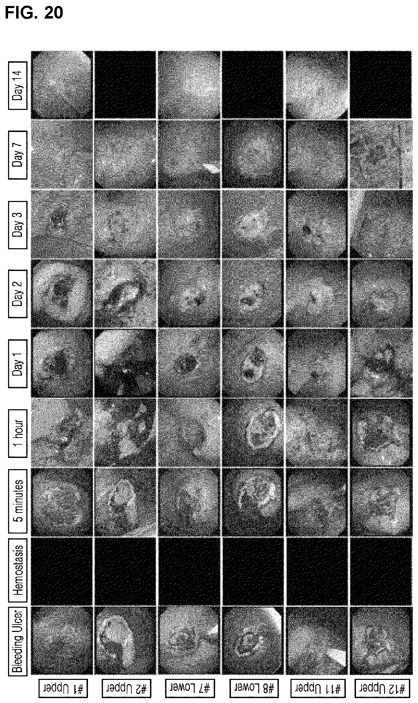

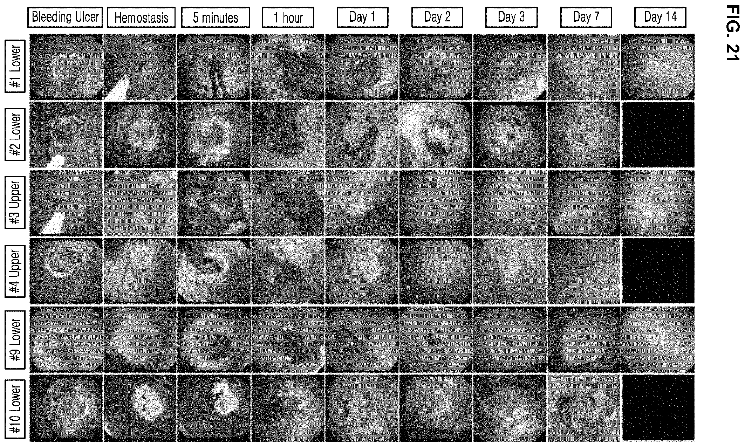

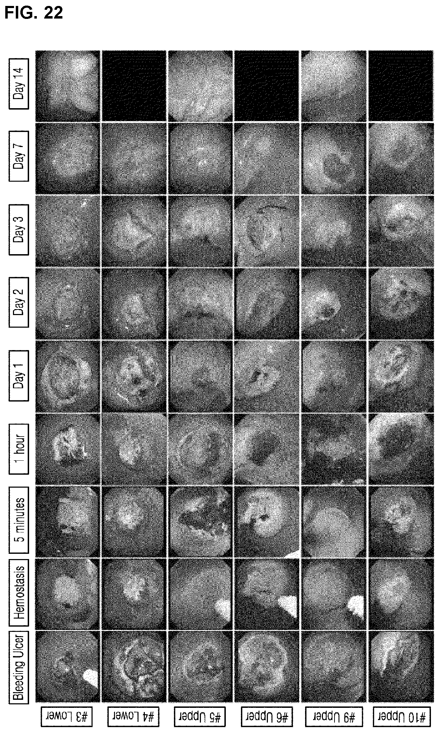

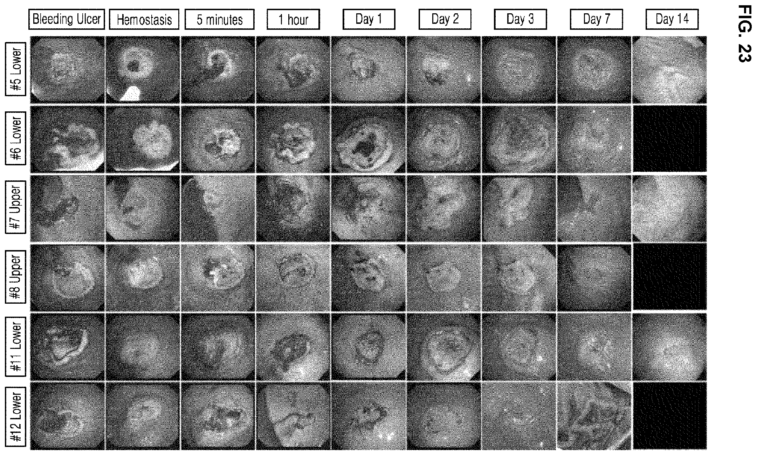

FIGS. 20 to 23 show endoscopic findings on the success rate of early hemostasis performed according to Experimental Example 3 of the present disclosure, where FIG. 20 shows experimental results of the No treatment group, FIG. 21 shows experimental results of the Endoclot treatment group, FIG. 22 shows experimental results of the CGGEL treatment group, and FIG. 23 shows experimental results of the CGGEL_Thrombin treatment group.

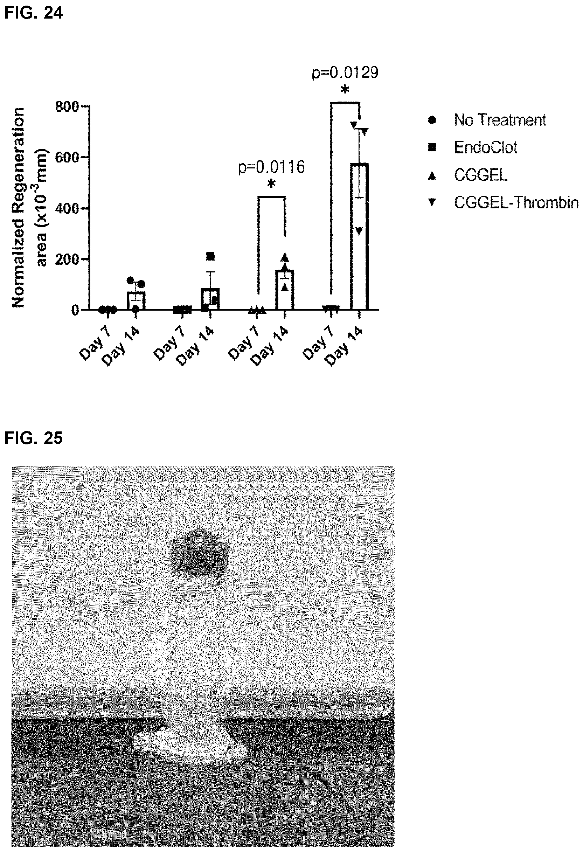

FIG. 24 shows results of an experiment on the mucosal regeneration area performed according to Experimental Example 3 of the present disclosure.

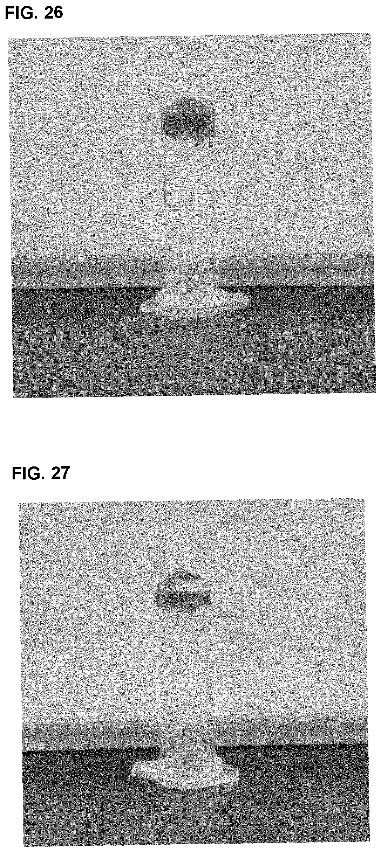

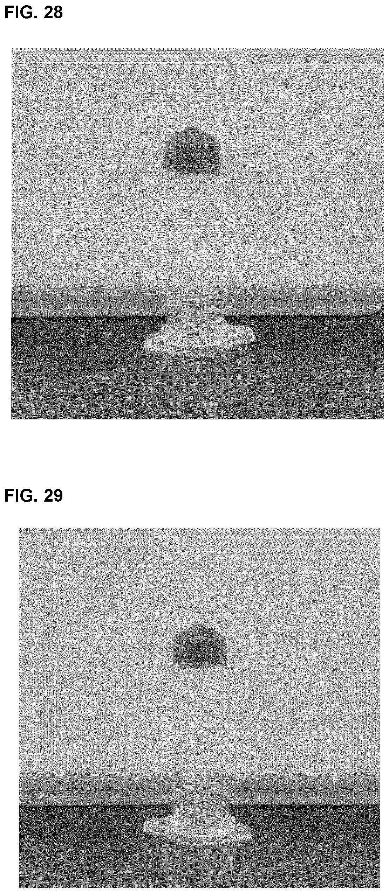

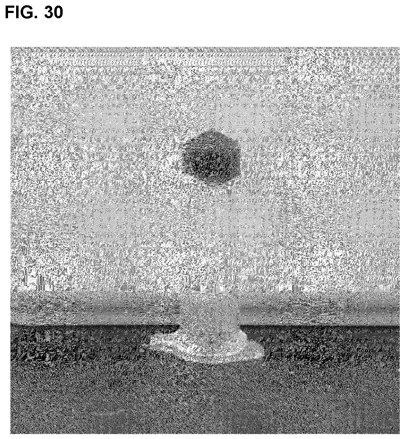

FIGS. 25 to 30 show results of blood clotting time measurement experiments performed according to Experimental Example 2 of the present disclosure, where FIG. 25 shows an experimental result performed on the sample S11-1, FIG. 26 shows an experimental result performed on the sample S12-1, FIG. 27 shows an experimental result performed on the sample S13-1, FIG. 28 shows an experimental result performed on the S14 sample, FIG. 29 shows an experimental result performed on the S15 sample, and FIG. 30 shows an experimental result performed on the S16 sample.

### [Mode for Disclosure]

The present disclosure provides a pharmaceutical composition that achieves hemostatic and wound treating effects more quickly in gastrointestinal ulcers and blocks the possibility of rebleeding, compared to conventionally developed and commercially available hemostatic agent compositions and wound healing agent compositions.

That is, the pharmaceutical composition for hemostasis in the gastrointestinal tract according to the present disclosure may include a first component composed of a water-soluble copolymer that is attached to gastrointestinal mucosal tissue, a second component composed of a water-absorbing material, and thrombin.

That is, the pharmaceutical composition for wound healing in the gastrointestinal tract according to the present disclosure may include a first component composed of a water-soluble copolymer that is attached to gastrointestinal mucosal tissue, a second component composed of a water-absorbing material, and thrombin.

The first component is attached to the gastrointestinal mucosal tissue to exert a physical hemostatic effect, and may include at least one of hydroxyethyl cellulose (HEC), sodium alginate, chitosan, hydroxypropyl cellulose (HPC), and polyethylene oxide (PEO).

The second component may include at least one of croscarmellose sodium (Ac-di-sol), sodium starch glycolate, calcium silicate, and crospovidone.

For example, the first component and the second component may be contained in an amount of 70 to 95 parts by weight: 5 to 30 parts by weight based on 100 parts by weight of the pre-composition before thrombin above is added. Within the above-described numerical range, a physical hemostatic effect may be shown by the protective wall formed by instantaneous gelation in the gastric mucosa, and at the same time, sufficient gel strength, for example, a gel strength of 0.001 MPa or more, may be developed. As an example, the first component and the second component may be contained in an amount of 70 to 90 parts by weight: 10 to 30 parts by weight based on 100 parts by weight of the pre-composition before thrombin above is added.

Thrombin above may include those extracted from bovine or pig plasma.

Thrombin above included in the pharmaceutical composition of the present disclosure can promote wound healing by regulating the expression of genes that perform inflammation and wound treating functions in monocytes, express tissue factor (TF) of playing various roles in the wound healing process through activation of protease-activated receptor (PAR), and accelerate the formation of new blood vessels by upregulating the expression of VEGF receptors in vascular endothelial cells.

In order to quickly achieve hemostatic and wound treating effects in gastrointestinal ulcers and prevent rebleeding phenomena from occurring, the inventors of the present disclosure found out that thrombin can exert hemostasis and wound treating effects in gastrointestinal ulcers as results of reviewing various substances with physiological hemostatic effects and repeating related experiments.

In addition, after repeated related experiments and trial and error with various hemostatic agent and/or wound healing agent compositions that can exert a synergistic effect with thrombin, the inventors of the present disclosure could confirm that the hemostatic and/or wound treating effects were improved when thrombin was mixed with the first component and the second component.

In addition, the inventors of the present disclosure could confirm that a synergistic effect of the pharmaceutical composition due to thrombin can be achieved when the first component, the second component, and thrombin are used in a specific composition range. Although the specific pharmacological mechanism has not yet been revealed, it was confirmed through experiments that the physical hemostatic effect of the first component and the second component and the physiological hemostatic effect of thrombin have a synergistic effect within the composition range of the pharmaceutical composition according to the present disclosure.

In order to confirm this, the inventors of the present disclosure conducted an experiment while putting thrombin into components of various commercially available types of pharmaceutical compositions for hemostasis and/or wound healing, and as a result, it was confirmed through experiments that, since a synergistic effect with thrombin is created when preparing a pharmaceutical composition of a powder form having a composition according to one embodiment of the present disclosure, firstly, the hemostatic and wound treating effects are superior to those of conventional commercially available hemostatic agents, secondly, the hemostatic and wound treating effects are excellent than when thrombin is mixed with other conventional commercially available hemostatic agents, and thirdly, the hemostasis and wound treating effects are excellent than when thrombin is used alone.

For example, thrombin above may be contained in the pharmaceutical composition in an amount of 20 IU/g to 10,000 IU/g. Within the above-described numerical range, the pharmaceutical composition may have additional hemostatic and wound treating effects. As an example, thrombin above may be included in the pharmaceutical composition in an amount of 100 IU/g to 5000 IU/g. Within the above-described numerical range, a synergistic effect of the pharmaceutical composition due to thrombin above can be achieved.

For example, thrombin above may be contained in an amount of 0.003 to 1.603 parts by weight per 100 parts by weight of the pharmaceutical composition. Within the above-described numerical range, the pharmaceutical composition may have additional hemostatic and wound treating effects. The higher the thrombin content, the more the short-term hemostatic effect may be improved, but the effect may converge at 1.603 parts by weight or more.

As an example, thrombin above may be contained in an amount of 0.016 parts by weight to 0.802 parts by weight per 100 parts by weight of the pharmaceutical composition. Within the above-described numerical range, the synergistic effect of the pharmaceutical composition due to thrombin above, that is, improved hemostatic and wound treating effects compared to the hemostatic and wound treating effects caused by thrombin itself, can be achieved.

Hereinafter, a configuration for defining the synergistic effect of the pharmaceutical composition due to thrombin above, that is, improved effects compared to the hemostatic and wound treating effects caused by thrombin itself, will be described.

When the time until blood does not flow after mixing 200 ul of blood and 15 mg of a sample of the pharmaceutical composition is measured as a blood clotting time, the pharmaceutical composition of the present disclosure can achieve a synergistic effect in which the blood clotting time appears shorter than a blood clotting time of the sample composed of thrombin above measured under the same conditions.

For example, the blood clotting time of the pharmaceutical composition performed according to one embodiment of the present disclosure may be less than 50 seconds, as an example, 30 seconds or less, and as a specific example, 25 seconds or less.

The pharmaceutical composition of the present disclosure can achieve a synergistic effect of hemostatic action in which the optical density (OD₅₄₀) at 540 nm measured by a method including steps of mixing 200 ul of blood and 15 mg of a sample of the pharmaceutical composition to react the mixture under conditions of 37°C and 10 minutes, and then adding 1 ml of purified water to stop a blood clotting reaction for 5 minutes, collecting a supernatant in which red blood cells hemolyzed by purified water excluding blood clots exist in the state that the blood clotting reaction is stopped, and adding 1 ml of purified water to the collected supernatant and thus collecting the remaining red blood cell hemolytic solution to measure OD₅₄₀ is shown to be lower than the OD₅₄₀ value of the sample composed of thrombin above measured under the same conditions.

For example, the optical density (OD₅₄₀) of the pharmaceutical composition performed according to one embodiment of the present disclosure may be less than 1.0, for example, 0.9 or less, for example, 0.8 or less, for example, 0.7 or less, and for specific example, 0.6 or less.

A synergistic effect can be achieved where the blood clot weights measured by a method comprising steps of mixing 200 ul of blood and 15 mg of a sample of the pharmaceutical composition to react the mixture under conditions of 37°C and 10 minutes, and then adding 1 ml of purified water to stop a blood clotting reaction for 5 minutes, separating coagulated blood clots in a state that the blood clotting reaction is stopped to fix them with 10% formalin for 20 minutes, and immersing the coagulated blood clots in purified water to wash them for 5 minutes and dry them for 24 hours, and then measuring their weights are shown to be heavier than the blood clot weight of a sample composed of thrombin above measured under the same conditions as described above.

For example, the blood clot weight of the pharmaceutical composition performed according to one embodiment of the present disclosure may be shown to be more than 8.0 mg, as an example, 10.0 mg or more, as an example, 15.0 mg or more, as an example, 20.0 mg or more, and as a specific example, 30.0 mg or more.

For example, the blood clot weight of the pharmaceutical composition performed according to one embodiment of the present disclosure may be shown to be 2 times or more, as an example 3 times or more, and as a specific example 4 times or more than the blood clot weight of the sample composed of thrombin above measured under the same conditions.

The pharmaceutical composition may further include a third component and/or a fourth component.

The pharmaceutical composition may further include the third component, which is a wound treating component.

For example, the third component may include at least one wound treating substance among an epidermal growth factor, a keratinocyte growth factor, and a basic fibroblast growth factor. The third component may be contained in a therapeutically useful amount, which may be easily determined by a person skilled in the art.

The pharmaceutical composition may further include the fourth component that is a component of improving the hemostatic effect.

For example, the fourth component may include at least one inorganic substance of calcium chloride, calcium phosphate, calcium hydroxide, calcium silicate, and calcium sulfate.

For example, the first component, the second component, and the fourth component may be contained at 40 to 75 parts by weight: 5 to 30 parts by weight: 10 to 40 parts by weight based on 100 parts by weight of the pre-composition before thrombin above is added, and may be contained at, as an example, 50 to 70 parts by weight: 10 to 20 parts by weight: 10 to 30 parts by weight. Within the above numerical range, the physical hemostatic effect and the hemostatic effect by appropriate configuration of chemical hemostatic factors may be improved.

The pharmaceutical composition of the present disclosure may include one in a powder form, and a pharmaceutical preparation formed from the pharmaceutical composition may include one in a powder form. At this time, the "powder form" includes all forms of powder, and may include, for example, pharmaceutical powder form and pharmaceutical granule form (i.e., granules) which are obtained through conventional formulation methods.

When the pharmaceutical composition and/or pharmaceutical preparation is prepared in a powder form, it makes it easy to pass through the catheter and apply by having appropriate fluidity, and may achieve additional effects of minimizing loss by forming a barrier while exhibiting high mucosal adhesion through instantaneous gelation at the application site.

The pharmaceutical composition of the present disclosure may be administered into the gastrointestinal tract through an endoscope (i.e., an endoscopic catheter) to perform hemostatic and wound treating actions in bleeding gastrointestinal ulcers.

Hereinafter, the present disclosure will be described in more detail through Examples. However, the Examples are for illustrating the present disclosure and the scope of the present disclosure is not limited to these Examples.

### Preparation Example

In order to confirm the hemostatic effect and wound treating effect of the pharmaceutical preparation according to the present disclosure, conventionally commercially available hemostatic agents and wound dressings were prepared as follows.

**[Table 1]**

| **Sample** | **S1** | **S2** | **S3** |
|---|---|---|---|
| **Name and item name** | Endoclot^{®} PHS absorbable in-body hemostatic products | Nexpowder^{™} Synthetic material absorbable wound dressing | CGGEL^{®} Synthetic material absorbable wound dressing |
| **Manufacturing company** | EndoClot Plus, Inc. | Next Biomedical Co., Ltd. | CG Bio Co., Ltd. |
| **Component** | Modified starch (100%) | -Succinic anhydride(ε-poly(L-lysine))-Aldehyded dextran-Polyvinylpyrrolidone | -Hydroxy ethyl cellulose |
| | | | -Sodium starch glycolate |
| | | | -Croscarmellose sodium |
| | | -Low-substituted | |
| | | Hydroxypropylcelluo se | -EGF |
| | | -Lactosse Monohydrate | |
| | | -Magnesium stearate | |

In addition, a powder preparation (S3') including 70 to 95 parts by weight of a mucoadhesive polymer and 5 to 30 parts by weight of a desiccant per 100 parts by weight of the pharmaceutical composition according to the above-described embodiment of the present disclosure was prepared.

### Preparing Example 1

In Preparing Example 1, as an example of the present disclosure, a composition in which 90 parts by weight of hydroxyethyl cellulose (HEC), 5 parts by weight of sodium starch glycolate, and 5 parts by weight of croscarmellose sodium were mixed was prepared as S3.

### Preparation Example 1

In S1, S2, and S3' according to Preparing Example 1, 0.080 parts by weight of a thrombin powder with high activity extracted from bovine plasma was injected into a glass vial based on the total weight of the pharmaceutical composition and mixed uniformly through a shaker to manufacture a sample in a powder form, and the composition containing the thrombin powder was indicated as '-1'. That is, S1-1, S2-1, and S3'-1 were manufactured, respectively, as compositions in a powder form so that S1-1 contains 0.080 parts by weight of thrombin in S1 based on the total weight of S1-1, S2-1 contains 0.080 parts by weight of thrombin in S2 based on the total weight of S2-1, and S3'-1 contains S3-1 0.080 parts by weight of thrombin in S3' based on the total weight of S3'-1.

At this time, the activity of thrombin injected was 624 IU/mg and the contents were controlled to become 500 IU/g.

In addition, a solution was prepared so as to have a concentration of 100 IU/ml by dissolving the thrombin solution sample (S4) in purified water using the same thrombin.

### Experimental Example 1

### Experimental Example 1-1: Measurement of blood clotting time

After mixing 200 ul of blood and 15 mg of each sample in a 2 ml microtube, the time until blood did not flow was measured as blood clotting time.

At this time, in the cases of S1-1, S2-1, and S3'-1, they were treated to become 7.5 IU of thrombin, and the thrombin content of each sample was controlled to be the same at 500 IU/g.

In addition, in the case of S4, 200 ul of blood and 75 ul of S4 sample were injected into a 2 ml microtube to measure blood clotting time, and it was treated to become 7.5 IU that is the same thrombin content in the same manner as in the cases of S1-1, S2-1, and S3'-1.

The experimental results are shown in Table 2 below and FIGS. 1 to 7 below.

**[Table 2]**

| **Sample** | **Blood clotting time (s)** |
|---|---|
| S1 | >300 |
| S1-1 | 45 |
| S2 | >300 |
| S2-1 | 53 |
| S3 | 200 |
| S3'-1 | 20 |
| S4 | 49 |

Referring to S1, S1-1, S2, S2-1, S3, S3'-1 in Table 2 and FIGS. 1 to 6, when thrombin is added to a conventional product, it could be confirmed that the hemostatic effect improves as the blood clotting time is shortened.

Meanwhile, when comparing (S1-1, FIG. 2), (S2-1, FIG. 4), and (S3'-1, FIG. 6), it could be confirmed that the pharmaceutical composition in which thrombin according to one embodiment of the present disclosure is mixed is shown to have a much shorter blood clotting time compared to the case of mixing thrombin with other compositions known as hemostatic agent components.

Additionally, when comparing (S3'-1, FIG. 6) and (S4, FIG. 7), the pharmaceutical composition (S3'-1) in which thrombin according to one embodiment of the present disclosure is mixed is shown to have a shorter blood clotting time than in the case (S4) of treating only the same amount of thrombin.

The reason why the synergistic effect of the blood clotting time of the pharmaceutical composition in which thrombin according to one embodiment of the present disclosure is mixed is shown to be shorter than that of thrombin itself at the same concentration as that of one embodiment of the present disclosure like this is expected that this is because thrombin, which directly acts on the physiological blood clotting mechanism to exert a hemostatic effect, shows a synergistic effect with the biocompatible polymer material of the pharmaceutical composition according to one embodiment of the present disclosure, which is composed of a hygroscopic polymer and a mucous membrane-adhesive polymer material and can produce a physical hemostatic effect by forming a barrier through a gelation reaction.

### Experimental Example 1-2: Evaluation of thrombus formation

To perform a blood clot weight and red blood cell hemolysis test, 200 µl of blood and 15 mg of each sample were mixed in a 2 ml microtube and reacted at 37°C for 10 minutes, and then 1 ml of purified water was added to stop the blood clotting reaction.

### Experimental Example 1-2-1: Measurement of red blood cell hemolysis degree

In a state that the blood clotting reaction was stopped after each sample was injected, a supernatant containing red blood cells hemolyzed with purified water, excluding blood clots, was collected.

Afterwards, 1 ml of purified water was added again, and the remaining red blood cell hemolysis solution was collected to measure OD₅₄₀. Here, OD₅₄₀ is the absorbance at 540 nm that can quantitatively evaluate hemolyzed red blood cells, and was measured using an ELISA device.

The experimental results are shown in Table 3 and FIG. 8.

**[Table 3]**

| Sample | OD₅₄₀ |
|---|---|
| S1-1 | 1.4756 |
| S2-1 | 0.9468 |
| S3'-1 | 0.5647 |
| S4 | 0.978 |

Referring to Table 3 and FIG. 8, when thrombin is added to conventional products, it has a level that is higher than (S2-1) or equivalent to (S1-1) a red blood cell hemolysis degree that is caused by thrombin itself, whereas it was confirmed that the pharmaceutical composition (S3'-1) in which thrombin is mixed according to one embodiment of the present disclosure showed a level of the red blood cell hemolysis degree that is a much lower not only compared to the conventional products but also than thrombin itself.

Accordingly, the pharmaceutical composition (S3'-1) in which thrombin is mixed according to one embodiment of the present disclosure has excellent early hemostatic effect within 5 minutes and has a rapid tissue regeneration effect, so it can be confirmed that the hemostatic effect and wound treating effect in bleeding gastrointestinal ulcers are excellent.

The reason why the synergistic effect appeared like this as in the results of blood clotting time evaluation, where the red blood cell hemolysis numerical value of the pharmaceutical composition according to one embodiment of the present disclosure was much lower than that of thrombin itself at the same concentration, is expected to be due to the fact that thrombin, which directly acts on the physiological blood clotting mechanism to show a hemostatic effect, shows a synergistic effect with the biocompatible polymer material of the pharmaceutical composition according to one embodiment of the present disclosure, which is composed of a hygroscopic polymer and a mucous membrane-adhesive polymer material and can show a physical hemostatic effect by forming a barrier through a gelation reaction.

### Experimental Example 1-2-2: Measurement of blood clot weight

In a state that the blood clotting reaction was stopped after each sample was injected, the coagulated blood clots were separated and fixed with 10% formalin for 20 minutes.

Thereafter, the coagulated blood clots were immersed in purified water, washed for 5 minutes, dried for 24 hours, and then weighed.

The results of the experiment above are shown in Table 4 below and FIGS. 9 to 12.

**[Table 4]**

| Sample | Blood clot weight (mg) |
|---|---|
| S1-1 | 3.1 |
| S2-1 | 3.9 |
| S3'-1 | 33.7 |
| S4 | 8.2 |

Referring to Table 4 and FIGS. 9 to 12, when thrombin is added to conventional products, the degree of early thrombus formation due to thrombin itself was shown to be low (S1-1, S2-1), whereas it was confirmed that the pharmaceutical composition (S3'-1) in which thrombin is mixed according to one embodiment of the present disclosure showed a much higher level of early thrombus formation degree than thrombin itself.

Accordingly, it can be confirmed that the pharmaceutical composition in which thrombin is mixed according to one embodiment of the present disclosure can quickly clot the blood at the wound site within 5 minutes, not only providing rapid hemostatic and wound treating effects, but also preventing rebleeding.

The reason why the synergistic effect of the pharmaceutical composition according to one embodiment of the present disclosure on the degree of early thrombus formation degree is much higher than that of thrombin itself is expected to be due to the fact that thrombin, which directly acts on the physiological blood clotting mechanism to show a hemostatic effect, can be composed of a hygroscopic polymer and a mucous membrane-adhesive polymer material to show a physical hemostatic effect by forming a barrier through a gelation reaction, and thrombin acts on hemostatic action before the gelation reaction of the polymer, resulting in a synergistic effect.

### Preparation Example 1-2

In S3' according to Preparing Example 1, 0.003 parts by weight, 0.016 parts by weight, 0.080 parts by weight, 0.401 parts by weight, 0.802 parts by weight, and 1.603 parts by weight of thrombin based on the total weight of the pharmaceutical composition were injected into each glass vial and mixed evenly to prepare samples in a powder form was prepared (S5 to S10). That is, the samples were prepared as compositions in a powder form, respectively, so that S5 contains 0.003 parts by weight of thrombin based on the total weight of S5, S6 contains 0.016 parts by weight of thrombin based on the total weight of S6, S7 contains 0.080 parts by weight of thrombin based on the total weight of S7, S8 contains 0.401 parts by weight of thrombin based on the total weight of S8, S9 contains 0.802 parts by weight of thrombin based on the total weight of S9, and S10 contains 1.603 parts by weight of thrombin based on the total weight of S10.

At this time, the amounts of thrombin injected are as shown in Table 5 below, and the same thrombin with 624 IU/mg activity was used in all S5 to S10.

### Experimental Example 1-3: Measurement of blood clotting time

After mixing 200 ul of blood and 15 mg of each sample in a 2 ml microtube, the time until blood did not flow was measured as blood clotting time. At this time, the blood clotting time (s) was measured three times, and then the average value was recorded.

The results of the experiment are shown in Table 5 below and FIGS. 13 to 19.

**[Table 5]**

| Sample | S5 | S6 | S7 | S8 | S9 | S10 |
|---|---|---|---|---|---|---|
| Thrombin (parts by weight) | 0.003 | 0.016 | 0.080 | 0.401 | 0.802 | 1.603 |
| Thrombin (IU/g) | 20 | 100 | 500 | 2500 | 5000 | 10000 |
| Blood clotting time (s) | 60 | 26 | 19 | 11 | 3 | 0 |

Referring to Table 5 and FIGS. 13 to 19, it was confirmed that the blood clotting time of the pharmaceutical composition according to one embodiment of the present disclosure shortened as the thrombin content increased.

In addition, it could be confirmed that when the thrombin content was 0.016 parts by weight or more based on the total weight compared to S4, the blood clotting time became significantly shorter than that caused by thrombin itself.

That is, when the thrombin content in the pharmaceutical composition of the present disclosure is 0.016 parts by weight or more based on the total weight, it could be confirmed that thrombin, which exhibits a physiological hemostatic effect, exhibits a synergistic effect with the biocompatible polymer material of the pharmaceutical composition according to one embodiment of the present disclosure, which exhibits a physical hemostatic effect.

### Preparing Example 2

In Preparing Example 2, compositions in which hydroxyethyl cellulose (HEC), sodium starch glycolate, and croscarmellose sodium are mixed in the composition ratios shown in Table 6 below were prepared as S11, S12, and S13 as an example of the present disclosure.

**[Table 6]**

| Sample | Hydroxy ethyl cellulose | Sodium starch glycolate | Croscarmellose sodium |
|---|---|---|---|
| S11 | 70 parts by weight | 15 parts by weight | 15 parts by weight |
| S12 | 80 parts by weight | 10 parts by weight | 10 parts by weight |
| S13 | 95 parts by weight | 2.5 parts by weight | 2.5 parts by weight |

### Production Example 2

In S11, S12, and S13 according to Preparing Example 2, thrombin powders with high activity extracted from bovine plasma were injected into a glass vial at the composition ratios shown in Table 7 based on the total weight of the pharmaceutical composition and mixed evenly using a shaker to prepare samples in a powder form and indicate the compositions containing thrombin powders as '-1'. That is, the samples were prepared as compositions in a powder form, respectively, so that S11-1 contains 0.080 parts by weight of thrombin in S11 based on the total weight of S11-1, S12-1 contains 0.401 parts by weight of thrombin in S12 based on the total weight of S12-1, and S13-1 contains 0.802 parts by weight of thrombin in S13 based on the total weight of S13-1.

At this time, the activity of injected thrombin was controlled to be the same at 624 IU/mg, and the content (IU/g) was controlled to be 500 for S11-1, 2500 for S12-1, and 5000 for S13-1.

In addition, thrombin solution samples S14, S15, and S16 had those with an activity of 624 IU/mg applied thereto and were prepared by dissolving them in purified water to become the concentrations shown in Table 8 below. Specifically, the thrombin solution samples were prepared so that the concentration (IU/ml) became 100 for S14, 500 for S15, and 1000 for S16.

**[Table 7]**

| Sample | Preparing Example 2 | Bovine Thrombin | Thrombin content (IU/g) |
|---|---|---|---|
| S11-1 | 99.920 parts by weight | 0.080 parts by weight | 500 |
| S12-1 | 99.599 parts by weight | 0.401 parts by weight | 2500 |
| S13-1 | 99.198 parts by weight | 0.802 parts by weight | 5000 |

**[Table 8]**

| Sample | Concentration (mg/ml) | Concentration (IU/ml) |
|---|---|---|
| S14 | 0.160 | 100 |
| S15 | 0.802 | 500 |
| S16 | 1.604 | 1000 |

### Experimental Example 2: Measurement of blood clotting time

After mixing 200 ul of blood and 15 mg of each sample in a 1.5 ml microtube, the time until blood did not flow was measured as blood clotting time. At this time, the blood clotting time (s) was measured three times and the average value was recorded. At this time, 75 ul of thrombin solution samples S14, S15, and S16 were put to proceed with the blood clotting reaction.

The results of the experiment are shown in Table 9 below and FIGS. 25 to 30.

**[Table 9]**

| Amount of thrombin treated in blood (IU) | 7.5 | | 37.5 | | 75 | |
|---|---|---|---|---|---|---|
| Sample | S11-1 | S14 | S12-1 | S15 | S13-1 | S16 |
| Blood clotting time | 40 | 51 | 20 | 25 | 7 | 8 |

Referring to Table 9 and FIGS. 25 to 30, it was confirmed that the blood clotting time of the pharmaceutical composition according to one embodiment of the present disclosure shortened as the thrombin content increased.

In addition, it could be confirmed that S11-1, S12-1, and S13-1 all showed shorter blood clotting times than when the blood was treated with the same amount of thrombin itself (S14, S15, S16) as the amount of thrombin treated with the blood in each Example.

That is, the powder preparations (S3', S5 to S10, S11, S12, S13) including 70 to 95 parts by weight of the mucoadhesive polymer and 5 to 30 parts by weight of the desiccant per 100 parts by weight of the pharmaceutical composition of the present disclosure could be confirmed to exhibit a synergistic effect with the biocompatible polymer material of the pharmaceutical composition according to one embodiment of the present disclosure, in which thrombin exhibiting a physiological hemostatic effect exhibits a physical hemostatic effect.

As such, it was confirmed that the pharmaceutical composition in a powder form according to one embodiment of the present disclosure was not only more excellent in the effect in all experiments than cases in which thrombin was added to other commercially available products, but also more excellent in the effect in all experiments than thrombin itself.

### Experimental Example 3: In-vivo test

The following in-vivo test was performed at Ajou University Hospital.

### <1> Test animal preparation and drug treatment

Twelve male pigs (Sus scrofa (porcine)) aged 4 to 5 months and weighing 35 to 40 kg were used in the test. In order for the pigs used in the experiment to have a bleeding tendency similar to that of humans, aspirin and heparin were administered as follows before and during the experiment.
① Antiplatelet drugs (aspirin, 200 mg/day) were crushed, mixed with food, and orally administered to pigs for 5 days before the experiment.
② For 3 days before the experiment, the minimum amount of feed (100 g/head) for antiplatelet drug administration was fed, and fasting was maintained on the day of the test.
③ Before the experiment, animal muscle relaxant and general anesthetic (zoletil+xylazine, 6:4) were mixed, and a mixture thereof was injected intramuscularly to provide pre-anesthesia, and then respiratory anesthesia was performed using isoflurane and a respiratory anesthesia machine.
④) Heparin (200 IU/kg) was administered intravenously immediately before the endoscopic procedure, and heparin was continuously administered using a syringe pump while the experiment was in progress (mixed with 10% glucose and administered at a rate of 10,000 IU/hr).

### <2> Hemorrhagic gastric ulcer model

A hemorrhagic gastric ulcer model was fabricated using the endoscopic mucosal resection (EMR) method in the following order.
① A double-channel upper endoscope (GIF-2T260M; Olympus Medical Systems, Tokyo, Japan) was inserted into the oral cavity of an anesthetized animal to enter the gastric cavity, and then a sizer with a diameter of 6 mm was attached to the gastric mucosal tissue.
② Based on the size of the sizer, the boundary of the area that would become a 20mm ulcer was marked using APC.
③ Isotonic saline was injected into the submucosal layer.
④ Mucosal resection was performed using endoscopic mucosal resection and exfoliation biopsy method (grasp-and-snare) methods.
   - The mucous membrane was pulled using forceps and captured with a snare.
   - The mucosal tissue was excised by passing an electric current through the snare.
⑤ If bleeding could not be induced, bleeding was induced by tearing away the exposed submucosal blood vessels using forceps.
⑥ Two ulcers were created in one pig using this method. One ulcer each was created in the upper and lower parts of the stomach, and spaced at least 5 cm apart so that they do not receive mutual influence.

### <3> Application of test substance

The samples S1, S3, and S3'-1 were prepared as test substances, and the administered dose was set at 3 g each.

According to the treatment protocol randomly distributed in advance, four types of treatment were performed: no treatment, Endoclot (S1 sample), CGGEL (S3 sample), and CGGEL_Thrombin (S3'-1 sample). In the case of powder spray treatment, the entire area of the ulcer was sprayed and enough was applied to make the basal surface of the ulcer invisible.

### <4> Measurement of evaluation variables

Archieve of hemostasis was defined as a case in which bleeding was not visually confirmed, and there was no visible outward flow of blood from an artificial ulcer.

In each artificial ulcer, bleeding was induced and whether there was hemostasis was checked for up to 5 minutes after application of the test substance, and the hemostasis state was checked again 1 hour later. Afterwards, animals were allowed to recover in individual cages but were not fed for 24 hours. Follow-up endoscopy for checking whether there was rebleeding at the artificial ulcer site and the treating status of the ulcer was performed 24 hours, 48 hours, 72 hours, 1 week, and 2 weeks after the procedure.

Whether there was rebleeding was determined by Forrest classification through endoscopic observation, and it was defined that rebleeding occurred in the case of endoscopic findings corresponding to Forrest classification I.

In addition, in order to evaluate the extent of ulcer treatment and the regenerative effect of the mucous membrane, changes in the area of the ulcer were recorded each time endoscopic observation was performed. A sizer of 6 mm to be a standard was placed on the ulcer and the area of the ulcer was measured (estimation). The area of the ulcer was measured and calculated through a Java-based image processing program (Image J, National Institutes of Health, USA).

Six pigs were observed 1 week after the procedure, and the remaining 6 pigs were observed until 2 weeks after the procedure, and then, at the end of the observation period, they were euthanized, and gastrointestinal tissues were removed. The removed tissue was fixed in 10% formalin solution and subjected to H&E staining and immunochemical staining (CD31). The specimen fixed in formalin solution was embedded in paraffin after passing through dehydration, transparent, and infiltration processes using an automatic tissue process. Afterwards, it was sectioned to a thickness of 4 µm using a rotary sectioner. The sectioned tissue was placed on a slide to fabricate an unstained slide. All tissues were deparaffinized and moisturization-treated, and one of them was subjected to H&E staining using Harris-Hematoxylin (YD Diagnostics, Korea) and eosin Y (Showa, Japan).

### <5> Sacrifice and specimen collection

Specimen collection from all animals was conducted after euthanasia according to the experimental schedule. To sacrifice experimental animals, pre-anesthesia was induced by intramuscular injection of 0.1 ml/kg of a 1:1 mixed solution of Zoletil and Xylazine, and then euthanasia was induced by intramuscular administration of 1 ml of succipharm. Afterwards, the transplant site of each subject was incised, and the gastrointestinal mucosa of the specimen-transplanted site was completely removed and fixed in a 10% neutral buffered formalin solution.

### <6> Experiment results

### Early hemostasis success rate

It was shown to be 0% (FIG. 20) in the No treatment group, it was shown to be 66.7% (FIG. 21) in the endoclot treatment group, and it was shown to be 16.7% (FIG. 22) in the CGGEL treatment group, whereas it was shown to be 100% (FIG. 23) in the CGGEL_Thrombin treatment group.

### Mucosal regeneration area

Referring to FIG. 24, the 14th day tissue showed significantly increased mucosal regeneration compared to the 7th day tissue in the CGGEL (EGF) treatment group and the CGGEL (Thrombin) treatment group. In particular, CGGEL (Thrombin) was shown to be the most excellent in the ulcer treatment effect by showing the results of increasing the mucous membrane regeneration area on day 14 in the CGGEL (Thrombin) treatment group approximately three times or more than the CGGEL (EGF) treatment group.

## Claims

1. A pharmaceutical composition for hemostasis in the gastrointestinal tract, comprising:
a first component composed of a water-soluble copolymer that is attached to gastrointestinal mucosal tissue;
a second component composed of a water-absorbing material; and
thrombin.

2. The pharmaceutical composition for hemostasis in the gastrointestinal tract of claim 1,
wherein the first component includes at least one of hydroxyethyl cellulose (HEC), sodium alginate, chitosan, hydroxypropyl cellulose (HPC), and polyethylene oxide (PEO),
the second component includes at least one of croscarmellose sodium (Ac-di-sol), sodium starch glycolate, calcium silicate, and crospovidone.

3. The pharmaceutical composition for hemostasis in the gastrointestinal tract of claim 1, wherein when the time until blood does not flow after mixing 200 ul of blood and 15 mg of a sample of the pharmaceutical composition is measured as a blood clotting time, the blood clotting time is shorter than a blood clotting time of the sample composed of thrombin above measured under the same conditions.

4. The pharmaceutical composition for hemostasis in the gastrointestinal tract of claim 3, wherein the optical density (OD₅₄₀) at 540 nm measured by a method comprising: steps of mixing 200 ul of blood and 15 mg of a sample of the pharmaceutical composition to react the mixture under conditions of 37°C and 10 minutes, and then adding 1 ml of purified water to stop a blood clotting reaction; collecting a supernatant in which red blood cells hemolyzed by purified water exist in the state that the blood clotting reaction is stopped; and adding 1 ml of purified water to the collected supernatant and thus collecting the remaining red blood cell hemolytic solution to measure OD₅₄₀ is lower than the OD₅₄₀ value of the sample composed of thrombin above measured under the same conditions.

5. The pharmaceutical composition for hemostasis in the gastrointestinal tract of claim 3, wherein the blood clot weights measured by a method comprising steps of: mixing 200 ul of blood and 15 mg of a sample of the pharmaceutical composition to react the mixture under conditions of 37°C and 10 minutes, and then adding 1 ml of purified water to stop a blood clotting reaction; separating coagulated blood clots in a state that the blood clotting reaction is stopped to fix them with 10% formalin for 20 minutes; and immersing the coagulated blood clots in purified water to wash them for 5 minutes and dry them for 24 hours, and then measuring their weights are heavier than the blood clot weight of a sample composed of thrombin above measured under the same conditions as described above.

6. The pharmaceutical composition for hemostasis in the gastrointestinal tract of claim 2, wherein the first component is composed of hydroxyethyl cellulose (HEC), and the second component is composed of sodium starch glycolate and croscarmellose sodium.

7. The pharmaceutical composition for hemostasis in the gastrointestinal tract of claim 6, wherein thrombin above is contained in an amount of 20 IU/g to 10,000 IU/g.

8. The pharmaceutical composition for hemostasis in the gastrointestinal tract of claim 1, further comprising a fourth component composed of at least one inorganic substance of calcium chloride, calcium phosphate, calcium hydroxide, calcium silicate, and calcium sulfate.

9. The pharmaceutical composition for hemostasis in the gastrointestinal tract of any one of claims 1 to 8, wherein the composition is in a powder form and is injected into the gastrointestinal tract to have a hemostatic action on bleeding gastrointestinal ulcers.

10. A pharmaceutical composition for wound healing in the gastrointestinal tract, comprising:
a first component composed of a water-soluble copolymer that is attached to gastrointestinal mucosal tissue;
a second component composed of a water-absorbing material; and
thrombin.

11. A pharmaceutical composition for hemostasis and wound healing in the gastrointestinal tract, comprising the composition according to any one of claims 1 to 8, wherein the composition is in a powder form and is injected into the gastrointestinal tract to have hemostatic and wound healing actions in bleeding gastrointestinal ulcers.
